(19) Europäisches Patentamt
European Patent Office
Office européen des brevets

(11) Veröffentlichungsnummer: **0 087 148**
**B1**

# (12) EUROPÄISCHE PATENTSCHRIFT

(45) Veröffentlichungstag der Patentschrift: **19.03.86**

(51) Int. Cl.⁴: **C 07 D 233/60**, A 01 N 43/50 // C07D303/22, C07D303/08, C07D303/04

(21) Anmeldenummer: **83101614.2**

(22) Anmeldetag: **02.05.81**

(60) Veröffentlichungsnummer der früheren Anmeldung nach Art. 76 EPÜ: **0040345**

(54) **1-Hydroxyethyl-azol-Derivate, Verfahren zu ihrer Herstellung sowie ihre Verwendung als Pflanzenwachstumsregulatoren und Fungizide.**

(30) Priorität: **16.05.80 DE 3018866**
**19.02.81 DE 3106076**

(43) Veröffentlichungstag der Anmeldung: **31.08.83 Patentblatt 83/35**

(45) Bekanntmachung des Hinweises auf die Patenterteilung: **19.03.86 Patentblatt 86/12**

(84) Benannte Vertragsstaaten: **AT BE CH DE FR GB IT LI NL SE**

(56) Entgegenhaltungen:
**EP - A - 0 054 974**
**EP - A - 0 057 357**
**EP - A - 0 061 051**
**EP - A - 0 061 835**
**DE - A - 2 618 979**
**US - A - 4 123 542**

**Die Akte enthält technische Angaben, die nach dem Eingang der Anmeldung eingereicht wurden und die *nicht in dieser Patentschrift enthalten sind.***

(73) Patentinhaber: **BAYER AG, Konzernverwaltung RP Patentabteilung, D-5090 Leverkusen 1 Bayerwerk (DE)**

(72) Erfinder: **Holmwood, Graham, Dr., Katernbergerstrasse 184, D-5600 Wuppertal 1 (DE)**
Erfinder: **Büchel, Karl Heinz, Prof.Dr., Dabringhausener Strasse 42, D-5093 Burscheid (DE)**
Erfinder: **Lürssen, Klaus, Dr., August-Kierspel-Strasse 89, D-5060 Bergisch-Gladbach 2 (DE)**
Erfinder: **Frohberger, Paul-Ernst, Dr., Willi-Baumeister-Strasse 5, D-5090 Leverkusen (DE)**
Erfinder: **Brandes, Wilhelm, Dr., Eichendorffstrasse 3, D-5653 Leichlingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 087 148 B1

LIBER, STOCKHOLM 1986

**Beschreibung**

Die Erfindung betrifft neue 1-Hydroxyethyl-azol-Derivate, ein Verfahren zu ihrer Herstellung sowie ihre Verwendung als Pflanzenwachstumsregulatoren und Fungizide.

Es ist bereits bekannt geworden, daß bestimmte 2-Halogen-ethyl-trialkyl-ammonium-halogenide pflanzenwachstumsregulierende Eigenschaften aufweisen (vgl. US-A - 3 156 554) So läßt sich z.B. mit Hilfe von 2-Chlorethyl-trimethyl-amnoniumchlorid eine Beeinflussung des Pflanzenwachstums, insbesondere eine Hemmung des vegetativen Pflanzenwachstums bei wichtigen Kulturpflanzen erzielen. Allerdings ist die Wirksamkeit dieses Stoffes, vor allem bei niedrigen Aufwandmengen, nicht immer ausreichend.

Es ist weiterhin bekannt, daß die 2-Chlorethylphosphon-äure eine Pflanzenwachstumsregulierende Wirkung aufweist (vgl. DE-A-1 667 968). Die mit dieser Substanz erzielten Ergebnisse sind jedoch ebenfalls nicht immer zufriedenstellend.

Ferner ist bereits bekannt geworden, daß Zink-ethylen-1,2-bisdithiocarbamidat ein gutes Mittel zur Bekämpfung von pilzlichen Pflanzenkrankweiten ist [ vgl Phytopathology 33, 1113 (1965)]. Jedoch ist dessen Einsatz nur beschränkt möglich, da es insbesondere bei niedrigen Aufwandmengen und Konzentrationen nicht immer befriedigend wirksam ist.

Aus der US-A-4 123 542 sind N-Alkyl-imidazole mit fungiziden Eigenschaften bekannt. Auch die Wirksamkeit dieser Stoffe ist aber bei niedrigen Dosierungen nicht in allen Fällen ausreichend.

Die EP-A-0 054 974 betrifft unter anderem auch solche 1-Hydroxyethyl-azol-Derivate, wie sie im vorliegenden Fall beansprucht werden. Die genannte Druckschrift basiert jedoch auf Anmeldungen, die erst nach dem Prioritätstag der vorliegenden Anmeldung eingereicht wurden.

Es wurden neue 1-Hydroxyethyl-azol-Derivate der allgemeinen Formel

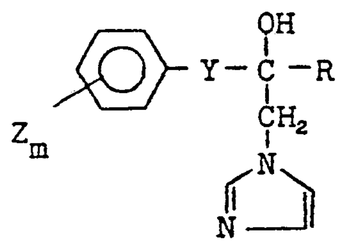

(I)

in welcher

R für tert.-Butyl, gegebenenfalls durch Methyl substituiertes Cyclopentyl oder für gegebenenfalls einfach oder zweifach, gleich oder verschieden durch Fluor, Chlor, Methyl oder Trifluormethyl substituiertes Phenyl steht,

Z für Fluor, Chlor, Brom, Methyl, tert.-Butyl, Cyclohexyl, Methoxy, Methylthio, Trifluormethyl, Trifluormethoxy, Trifluormethylthio oder für jeweils gegebenenfalls einfach oder zweifach, gleich oder verschieden durch Fluor, Chlor und/oder Methyl substituiertes Phenyl, Phenoxy, Benzyl oder Benzyloxy steht,

m für die Zahlen 0, 1 oder 2 steht und

Y für die Gruppierungen $-CH_2-CH_2-$ und $-O-CH_2-$ steht, wobei im Falle der $-OCH_2$-Gruppe das Sauerstoffatom mit dem Phenylrest verbunden ist

sowie deren Säureadditions-Salze und Metallsalz-Komplexe gefunden.

Die Verbindungen der Formel (I) besitzen ein asymmetrisches Kohlenstoffatom und können deshalb in den beiden optischen Isomerenformen anfallen. Die Erfindung betrifft sowohl die Isomerengemische als auch die einzelnen Isomeren.

Weiterhin wurde gefunden, daß man die 1-Hydroxyethyl-azol-Derivate der Formel (I) erhält, wenn man Oxirane der Formel

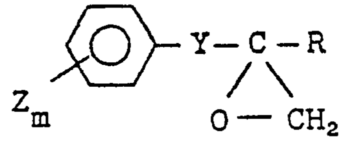

(II)

in welcher
R, Y, Z und m die oben angegebene Bedeutung haben, mit Imidazol der Formel

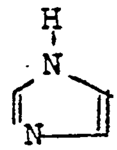

(III)

in Gegenwart eines Verdünnungsmittels und gegebenenfalls in Gegenwart einer Base unsetzt, und gegebenen alls anschließend an die erhaltenen Verbindungen der Formel (I) eine Säure oder ein Metallsalz addiert.

Außerdem wurde gefunden, daß die neuen 1-Hydroxyethyl-azol-Derivate der Formel (I) starke pflanzenwachstumsregulierende und starke fungizide Eigenschaften besitzen.

Ueberraschenderweise zeigen die erfind xyethyl-azol-Derivate der Formel (I) eine bessere pflanzenwachstumsregulierende Wirkung als das bekannte 2-Chlorethyl-trimethylammoniumchlorid und als die eben alls bekannte 2-Chlorethylphosphonsäure, welches anerkannt gut wirksame Stoffe gleicher Wirkungsart sind. Außerdem besitzen die erfindungsgemäßen Verbindungen überraschenderweise eine bessere fungizide Wirkung, als das aus dem Stand der Technik bekannte Zink-ethylen-1,2-bisdithiocarbamidat, welches eine wirkungsmäßig naheliegende Verbindung ist.

Schließlich übertreffen die erfindungsgemäßen Stoffe überraschenderweise auch die konstitutionell ähnlichsten Verbindungen, die aus der US-A-4 123 542, der DE-A-2-734 426 und der US-A-4 381 306 bekannt sind, bezüglich ihrer pflanzenwuchsregulierenden und fungiziden Wirksamkeit.

Die erfindungsgemäßen 1-Hydroxyethyl-azol-Derivate sind durch die Formel (I) allgemein definiert. Sie unterscheiden sich von den in der US-A-4 123 542 beschriebenen Verbindungen durch die Substituenten, die an dem Kohlenstoffatom in 2-Stellung zum Imidazol-Rest enthalten sind.

Im einzelnen seien außer den bei den Herstellungsbeispielen genannten Verbindungen folgende Verbindungen der allgemeinen Formel (I) genannt:

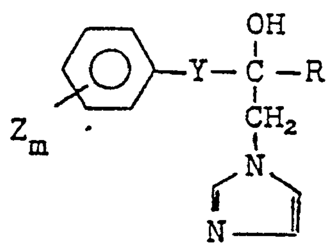

(I)

Tabelle 1

| $Z_m$ | Y | R |
|---|---|---|
| 4- 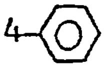 | $-O-CH_2-$ | $-C(CH_3)_3$ |
| 4- 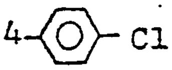 | " | " |
| 4-O- 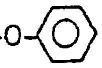 | " | " |
| 4-O- 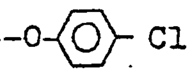 | " | " |
| $4-CH_2-$ 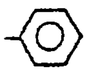 | " | " |
| $4-CH_2-$ 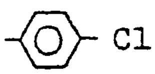 | " | " |
| $4-O-CH_2-$ 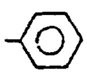 | " | " |
| $4-O-CH_2-$ 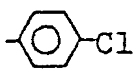 | " | " |
| $3,4-Cl_2$ | " | " |
| $4-CF_3$ | " | " |
| $4-OCF_3$ | " | " |
| $4-SCF_3$ | " | " |
| $4-SCH_3$ | " | " |
| $4-C(CH_3)_3$ | " | " |

Tabelle 1 (Fortsetzung)

| $Z_m$ | Y | R |
|---|---|---|
| 4- 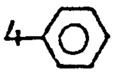 | $-O-CH_2$ | 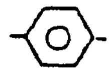 -Cl |
| 4- 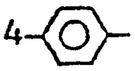 Cl | " | " |
| 4-O- 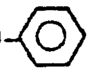 | " | " |
| 4-O- 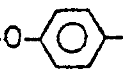 Cl | " | " |
| $4-CH_2$ 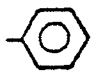 | " | " |
| $4-CH_2$ 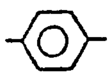 Cl | " | " |
| $4-O-CH_2$ 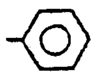 | " | " |
| $4-O-CH_2$ 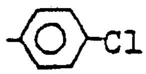 Cl | " | " |
| $3,4-Cl_2$ | " | " |
| $4-CF_3$ | " | " |
| $4-OCF_3$ | " | " |
| $4-SCF_3$ | " | " |
| $4-SCH_3$ | " | " |
| $4-C(CH_3)_3$ | " | " |

Tabelle 1 (Fortsetzung)

| $Z_m$ | Y | R |
|---|---|---|
| 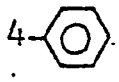 | $-CH_2-CH_2-$ | $-C(CH_3)_3$ |
| 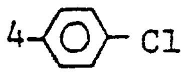 | " | " |
| 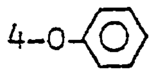 | " | " |
| 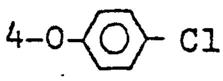 | " | " |
| 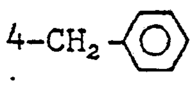 | " | " |
| 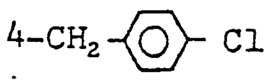 | " | " |
| 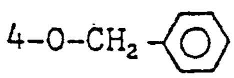 | " | " |
| 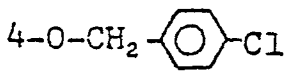 | " | " |
| 3,4-$Cl_2$ | " | " |
| 4-$CF_3$ | " | " |
| 4-$OCF_3$ | " | " |
| 4-$SCF_3$ | " | " |
| 4-$SCH_3$ | " | " |
| 4-$C(CH_3)_3$ | " | " |

Tabelle 1 (Fortsetzung)

| $Z_m$ | Y | R |
|---|---|---|
| 4-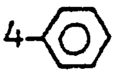 | $-CH_2-CH_2-$ | 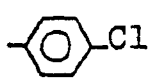 |
| 4-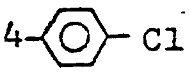 | " | " |
| 4-O-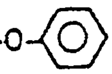 | " | " |
| 4-O-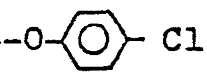 | " | " |
| $4-CH_2-$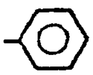 | " | " |
| $4-CH_2-$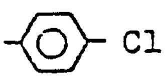 | " | " |
| $4-O-CH_2-$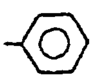 | " | " |
| $4-O-CH_2-$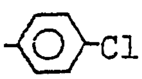 | " | " |
| $3,4-Cl_2$ | " | " |
| $4-CF_3$ | " | " |
| $4-OCF_3$ | " | " |
| $4-SCF_3$ | " | " |
| $4-SCH_3$ | " | " |
| $4-C(CH_3)_3$ | " | " |

Tabelle 1 (Fortsetzung)

| $Z_m$ | Y | R |
|---|---|---|
| $2,4-Cl_2$ | $-CH_2-CH_2-$ | $-C(CH_3)_3$ |
| $4-CH_3$ | " | " |
| $4-Cl, 2-CH_3$ | " | " |
| $4-F$ | $-O-CH_2-$ | $-C(CH_3)_3$ |
| $2-CH_3$ | $-CH_2-CH_2-$ | $-C(CH_3)_3$ |

Verwendet man beispielsweise 2-(4-Chlorphenoxy-methyl)-2-tert.-butyl-oxiran und-Imidazol als Ausgangsstoffe, so kann der Ablauf des erfindungsgemäßen Verfahrens durch das folgende Formelschema wiedergegeben werden:

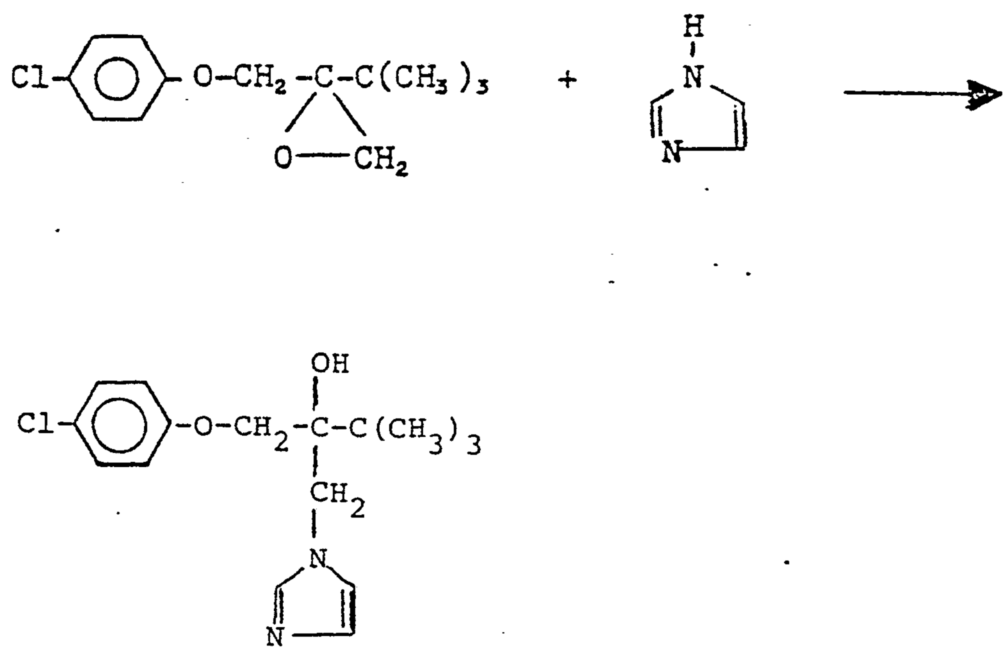

Die bei der Durchführung des erfindungsgemäßen Verfahrens als Ausgangsstoffe zu verwendenden Oxirane sind durch die Formel (II) allgemein definiert. In dieser

Formel haben R, Y, Z und der Index 3 die Bedeutungen, die bereits im Zusammenhang mit der Beschreibung der erfindungsgemäßen Stoffe der Formel (I) für diese Substituenten bzw. für den Index m genannt wurden.

Die Oxirane der Formel (II) sind zum Teil neu.

Die Oxirane der Formel (II) lassen sich herstellen, indem man Retone der Formel

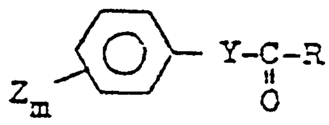 (IV)

in welcher
R, Y, Z und m die oben angebene Bedeutung haben, entweder
α) mit Dimethyloxosulfonium-methylid der Formel
δ + δ
$(OH_3)_2SOCH_2$ (V)
in Gegenwart eines Verdünnungsmittels umsetzt,
β) mit Trimethylsulfonium-methylsulfat der-Formel oder

$$\left[ (CH_3)_3\overset{(+)}{S} \right] CH_3SO_4^{(-)} \qquad (VI)$$

in Gegenwart eines inerten organischen Lösungsmittels sowie in Gegenwart einer Base umsetzt.

Die bei der Herstellung der Oxirane der Formel (II) als Ausgangsstoffe benötigten Ketone der Formel (IV) sind bekannt [vgl. DE-C- 22 01 063, DE-A- 27 05 678, DE-A- 27 37 489, Tetrahedron 31, 3 (1975) und Chemical Abstracts 84, 73 906 n] oder lassen sich nach im Prinzip bekannten Verfahren herstellen.

Das bei der Verfahrensvariante (α) benötigte Dimethyloxosulfonium-methylid der Formel (V) ist ebenfalls bekannt [vgl. J. Amer. Chem. Soc. 87, 1363-1364 (1965)]. Es wird bei der obigen Umsetzung in frisch zubereitetem Zustand verarbeitet, indem man es in situ durch Umsetzung von Trimethyloxosulfoniumjodid mit Natriumhydrid bzw. Natriumamid in Gegenwart eines Verdünnungsmittels erzeugt.

Das bei der Verfahrensvariante (β) benötigte Trimethylsulfonium-methylsulfat der Formel (VI) ist ebenfalle bekannt [vgl. Heterocycles 8, 397 (1977)]. Es wird bei der obigen Umsetzung ebenfalls in frisch hergestelltem Zustand eingesetzt, indem man es durch Reaktion von Dimethylsulfid mit Dimethylsulfat in situ erzeugt.

Bei der Variante (α) des Verfahrens zur Herstellung der Oxirane der Formel (II) kommt als Verdünnungsmittel vorzugsweise Dimethylsulfoxid in Frage.

Die Reaktionstemperaturen können bei der oben beschriebenen Verfahrensvariante (α) innerhalb eines größeren Bereiches variiert werden. Im allgemeinen arbeitet man bei Temperaturen zwischen 20 und 80°C.

Die Durchführung des Verfahrens zur Herstellung der Oxirane der Formel (II) nach der Variante (α) und die Aufarbeitung des bei dieser Synthese anfallenden Reaktionsgemisches erfolgen nach üblichen Methoden [vgl. J. Amer. Chem. Soc. 87, 1363-1364 (1965)].

Bei der Variante (8) zur Herstellung der Oxirane der Formel (II) kommt als inertes organisches Lösungsmittel vorzugsweise Acetonitril in Betracht.

Als Basen können bei der Varfahrensvariante (8) starke anorganische oder organische Basen verwendet werden. Vorzugsweise in Frage kommt Natriummethylat.

Die Reaktionstemperaturen können bei der oben beschriebenen Verfahrensvariante (8) innerhalb eines bestimmten Bereiches variiert werden. Im allgemeinen arbeitet man bei Temperaturen zwischen 0 und 60°C, vorzugsweise bei Raumtemperatur.

Die Durchführung des Verfahrens zur Herstellung der Oxirane der Formel (II) nach der Variante (8) und die Aufarbeitung des bei dieser Synthese anfallenden Reaktionsproduktes erfolgen nach üblichen Methoden [vgl. Heterocycles 8, 397 (1977)].

Die Oxirane der Formel (II) können bei dem erfindungs-gemäßen Verfahren gegebenenfalls ohne Isolierung direkt weiter umgesetzt werden.

Das außerdem für das erfindungsgemäße Verfahren als Ausgangsstoff zu verwendende Imidazol. der Formel (III) ist bekannt.

Als Verdünnungsmittel kommen für die erfindungsgemäße Umsetzung alle inerten organischen Lösungsmittel infrage. Hierzu gehören vorzugsweise Alkohole, wie z.B. Ethanol und Methoxyethanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für die erfindungsgemäße Umsetzung alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kaliummethylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens in einem

größeren Bereich variiert werden. Im allgemeinen arbeitet man bei Temperaturen zwischen C und 200°C, vorzugsweise zwischen 60 und 150°C.

Die erfindungsgemäße Umsetzung kann gegebenenfalls unter erhöhtem Druck durchgeführt werden. Im allgemeinen arbeitet man zwischen 1 und 50 bar, vorzugsweise zwischen 1 und 25 bar.

Bei der Durchführung des erfindungsgemäßen Verfahrens setzt man auf 1 Mol Oxiran der Formel (II) vorzugsweise 1 bis 2 Mol Imidazol und gegebenenfalls 1 bis 2 Mol Base ein. Die Isolierung der Endprodukte erfolgt in allgemein üblicher Weise.

Die nach dem erfindungsgemäßen Verfahren erhältlichen Verbindungen der Formel (I) können in Säureadditions-Salze bzw. Metallsalz-Komplexe überführt werden.

Zur Herstellung von physiologisch verträglichen Säureadditionssalzen der Verbindungen der Formel (I) kommen vorzugsweise folgende Säuren in Frage: Die Halogenwasserstoffsäuren, wie z.B. Chlorwasserstoffsäure und die Bromwasserstoffsäure, insbesondere die Chlorwasserstoffsäure, ferner Phosphorsäure, Salpetersäure, Schwefelsäure, monound bifunktionelle Carbonsäuren und Hydroxycarbonsäuren, wie z.B. Essigsäure, Maleinsäure, Bernsteinsäure, Fumarsaure, Weinsäure, Citronensäure, Salicylsäure, Sorbinsäure, Milchsäure sowie Sulfonsäuren, wie z.B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösung einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Hauptgruppe und der I. und II. sowie IV. bis VIII. Nebengruppe des Periodensystems in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metall-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisieren reinigen.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anmendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesatz werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuches von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hiedurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalle dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegrtativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums ksnn aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsrl erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an

Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.ß. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflsnzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseita kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee ains vollständige machanische oder manuelle Beerntung in einem Arbeitsgang vorgenommen werden kann.

Durch Anwendung von Wachtumsregulatoren kann ferner die Samen- oder Knospenruhe der Planzen beeinflußt werden, sodaß die Pflanzen, wie z.B. Ananas oder Zierpflanzen in Gärtnereien zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermieden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweisen ungeeignet sind.

Die erfindungsgemäßen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygcmycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäß verwendbaren Wirkstoffe mit besonders gutem Erfolg zur Bekämpfung solcher Pilze eingesetzt werden, die echte Hehltauerkrankungen hervorrufen; so zur Bekämpfung von Erysiphe-Arten, wie z.B. gegen den Erreger des Gersten- bzw. des Getreidemehltaus (Erysiphe graminis).

Besonders hervorzuheben ist, daß die erfindungsge-mäßen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Ssatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und / oder festen Trägerstoffen, gegebenenfalls unter Verwendung von oberflächenaktiven Mitteln, also Emulgiermitteln und / oder Dispergiermitteln und / oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte

Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und / oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Hangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird,dessen genaue Abgrenzung sich nach den klimatischen und vegetativen Gegebenheiten richtet.

Auch beim Einsatz der erfindungsgemäßen Stoffe als Fungizide kann die Aufwandmenge je nach Art der Applikation in einem größren Bereich varriiert werden. So liegen die Wirkstoffkonzentrationen bei der Behandlung von Pflanzenteilen in den An wendungsform en im allgemeinen zwischen 1 und 0,0001 Gew.-%, vorzugsweise zwischen 0,5 und 0 001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

**Herstellungsbeispiele**

**Beispiel 1**

(I-1)

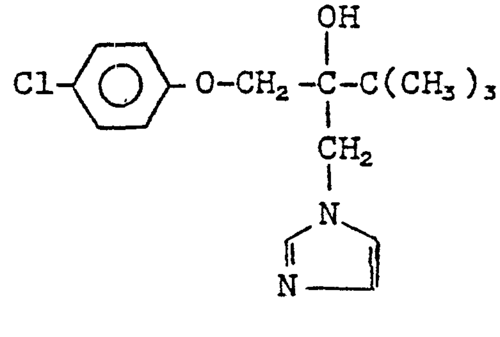

2,71 g (0,1178 Mol) Natrium in 250 ml absolutem Ethanol werden mit 8,02 g (0,1178 Mol) Imidazol versetzt. Innerhalb von 30 Minuten läßt man bei Raumtemperatur eine Lösung von 14,17 g (0,0589 Mol) 2-(4-Chlorphenoxy-methyl)-2-tert.-butyl-oxiran in 100 ml Ethanol zutropfen. Danach wird das Reaktionsgemisch 8 Stunden unter Rückfluß erhitzt, eingeengt und der Rückstand in Ether aufgenommen. Man extrahiert dreimal mit 1 n Salzsäure, neutralisiert die vereinigten Salzsäurephasen mit Natriumhydrogencarhonat und extrahiert anschließend mit Essigester. Nach dem Einengen und Umkristallisieren aus Cyclohexan erhält man 11,6 g (64 % der Theorie) 2-(4-Chlorphenoxy-methyl)-3,3-dimethyl-1-(imidazol-1-yl)-butan-2 ol vom Schmelzpunkt 154 - 55° C.

**Herstellung des Ausgangsproduktes**

(II-1) 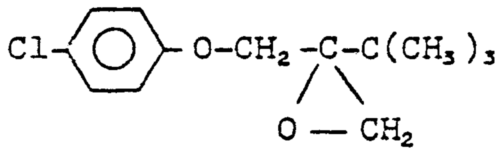

Eine Lösung von 189 ml (2,0 Mo1) Dimethylsulfat in 1200-ml absolutem Acetonitril wird bei Raumtemperatur mit einer Lösung von 162 ml (2,2 Mo1) Dimethylsulfid in 400 ml absolutem Acetonitril versetzt. Man läß die Reaktionsmischung über Nacht bei Raumtemperatur rüen Danach wehden 118.8 g (2,2 Mo1) Natriummethylat zugegeben. Man läßt 30 Minuten rühren und versetzt dann innerhalb von 30 Minuten tropfenweise mit einer Lösung von 272 g (1,2 Mo1) 1-(4-Chlorphenoxy)-3,3-di-methyl-butan-2-on in 600 ml absolutem Acetonitril. Man läßt das Reaktionsgemisch über Nacht nachrühren. An-schließend wird eingeengt, der Rückstand zwischen Wasser und Essigester verteilt, die organische Phase abgetrennt, zweimal mit Wasser und einmal mit gesättigter Natriumchloridlösung gewaschen, über Natriumsulfat getrocknet, eingeengt und der Rückstand im Vakuum destilliert. Man erhält 242,4 g (84% der Theorie) 2-(4-Chlorphenoxy-methyl)-2-tert.-butyl—oxiran vom Siedepunkt 115-22°C/0,003 mm Hg-Säule und vom Schmelzpunkt 50-52°C.

In analoger Weise wurden die nachfolgenden Verbindungen der allgemeinen Formel (I) erhalten:

Tabelle 2:

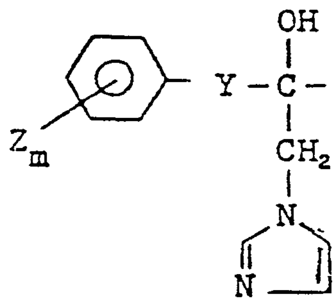

(I)

| Bsp. Nr. | $Z_m$ | Y | R | Schmelzpunkt (°C) |
|---|---|---|---|---|
| I - 2 | 2,4-$Cl_2$ | $-O-CH_2-$ | $-C(CH_3)_3$ | 152-54 |
| I - 3 | 4-$CH_3$ | $-O-CH_2-$ | $-C(CH_3)_3$ | 129-31 |
| I - 4 | 2-$CH_3$ | $-O-CH_2-$ | $-C(CH_3)_3$ | 123-24 |
| I - 5 | 4-Cl, 2-$CH_3$ | $-O-CH_2-$ | $-C(CH_3)_3$ | 157-59 |
| I - 6 | 4-Cl | $-CH_2-CH_2-$ | $-C(CH_3)_3$ | 157,5-59,5 |
| I - 7 | 4-F | $-CH_2-CH_2-$ | $-C(CH_3)_3$ | 124-25 |
| I - 8 | 2-$CH_3$ | $-CH_2-CH_2-$ | $-C(CH_3)_3$ | 94-99 |
| I - 9 | 4-Cl | $-O-CH_2-$ | 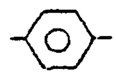 Cl | 216-17 (x 1/2 NDS)* |

*NDS = 1,5-Naphthalindisulfonsäure

| Bsp. Nr. | $Z_m$ | Y | R | Schmelzpunkt (°C) |
|---|---|---|---|---|
| I-10 | 2,4-$Cl_2$ | $-CH_2-CH_2-$ | $-C(CH_3)_3$ | 118-19 |
| I-11 | 4-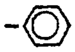 | $-O-CH_2-$ | " | 169-70,5 |
| I-12 | 2-Cl | $-O-CH_2-$ | " | 122-24 |
| I-13 | 4-Cl | $-O-CH_2-$ | 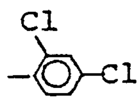 | 134-35,5 |
| I-14 | 4-F | $-O-CH_2-$ | $-C(CH_3)_3$ | 141-42 |
| I-15 | 3-Cl | $-O-CH_2-$ | " | 124 |
| I-16 | 2-Cl, 4-F | $-O-CH_2-$ | " | 137 |
| I-17 | 4-$CH_3$ | $-CH_2-CH_2-$ | " | 101-03 |
| I-18 | 4-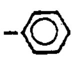-Cl | $-O-CH_2-$ | " | 174-76 |

Entsprechend Beispiel 1 -werden die nachfolgenden Zwischenprodukte der allgemeinen Formel (11) erhalten:

Tabelle 3:

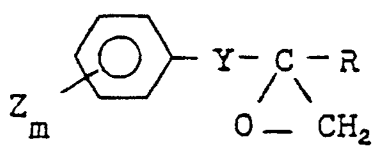

(II)

| Bsp. Nr. | $Z_m$ | Y | R | Siedepunkt(°C)/ mm Hg-Säule |
|---|---|---|---|---|
| II- 2 | 2,4-$Cl_2$ | $-O-CH_2-$ | $-C(CH_3)_3$ | 125-27/0,3 |
| II- 3 | 4-$CH_3$ | $-O-CH_2-$ | " | 85/0,07 |
| II- 4 | 2-$CH_3$ | $-O-CH_2-$ | " | 89/0,07 |
| II- 5 | 4-Cl,2-$CH_3$ | $-O-CH_2-$ | " | 114-17/0,33 |
| II- 6 | 4-Cl | $-CH_2-CH_2-$ | " | 99-103/0,005 |
| II- 7 | 2,4-$Cl_2$ | $-CH_2-CH_2-$ | " | 79/0,004 |
| II- 8 | 4-F | $-CH_2-CH_2-$ | " | 79-89/0,003 |
| II- 9 | 4-$CH_3$ | $-CH_2-CH_2-$ | " | 74-78/0,003 |
| II-10 | 2-$CH_3$ | $-CH_2-CH_2-$ | " | 95/0,005 |

Die gute Wirksamkeit der erfindungsgemäßen Stoffe geht aus den nachfolgenden Beispielen hervor. In diesen Beispielen werden die nachstehend angegebenen Verbindungen als Vergleichssubstanzen eingesetzt:

(A) = 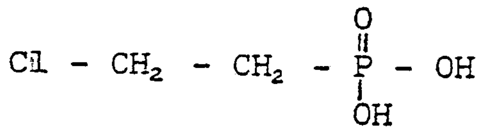

2-Chlorethylphosonsäure (B) = $C1-CH_2-CH_2-N^{\oplus}(CH_3)_3 \cdot Cl^{\ominus}$
2-Chlorethyl-trimethyl-ammoniumchlorid

(C) = 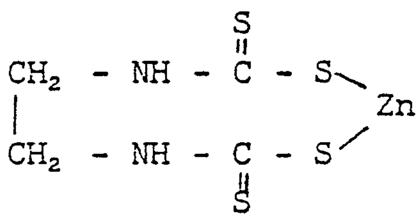

Zink-ethylen-1,2-bisdithiocarbamidat

**Beispiel A**

**Wuchshemmung bei Zuckerrüben**

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchshemmung in Prozent des Zuwachses der Kontrollpflanzen berechnet. Es bedeuten 0 % Wuchshemmung ein Wachstum entsprechend dem der Kontrollpflanzen. 100 % Wuchshemmung bedeutet Stillstand des Wachstums.

Die erfindungsgemäßen Wirkstoffe zeigen in diesem Test eine sehr gute Wuchshemmung.

**Beispiel B**

**Wuchshemmung bei Sojabohnen**

Lösungsmittel: 10 Gewichtsteile Methanol
Emulgator: 2 Gewichtsteile Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Junge Sojabohnenpflanzen werden in dem Stadium, in dem die ersten Folgeblätter entfaltet sind, mit den Wirkstoffzubereitungen tropfnaß besprüht. Nach 2 Wochen wird der Zuwachs gemessen und die Wuchshemmung in % des Zuwachses der Kontrollpflanzen berechnet. Es bedeuten 100 % den Stillstand des Wachstums und 0 % ein Wachstum entsprechend dem der unbehandelten Kontrollpflanzen.

Die erfindungsgemäßen Wirkstoffe zeigen in diesem Test eine sehr gute Wuchshemmung.

**Beispiel C**

**Wuchshemmung bei Baumwolle**

Lösungsmittel: 30 Gewichtsteile Dimethylformamid
Emulgator: 1 Gewichtsteile Polyoxyethylen-Sorbitan-Monolaurat

Zur Herstellung einer zweckmäßigen Wirkstoffzubereitung vermischt man 1 Gewichtsteil Wirkstoff mit der angegebenen Menge Lösungsmittel und Emulgator und füllt mit wasser auf die gewünschte Konzentration auf.

Baumwollpflanzen werden im Gewächshaus bis zur vollen Entfaltung des 5.Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Die erfindungsgemäßen Wirkstoffe zeigen in diesem Test eine sehr gute Wuchshemmung.

**Beispiel D**

**Stimulierung der $CO_2$-Assimilation bei Sojabohnen**

Sojabohnen werden wie im Beispiel (B) beschrieben, mit den Wirkstoffzubereitungen behandelt. 7 Tage nach der Behandlung wird an Blattscheiben dieser Pflanzen und entsprechender Kontrollpflanzen die $CO_2$-Assimilation mit Hilfe eines Infrarotanalysators gemessen. Die erfindungsgemäßen Wirkstoffe(I-8) und (I-11) zeigen in den Konzentrationen 2-50, 500 und 1000 ppm eine gegenüber den Kontrollen deutlich erhöhte $CO_2$-Assimilation. Dieser Effekt läßt Ertragssteigerungen durch den Wirkstoff erwarten.

**Beispeil E**

**Erysiphe-Test (Gerste) /protektiv**

Lösungsmittel: 100 Gewichtsteile Dimethylformamid
Emulgator: 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Ueberlegenheit in der Wirksamkeit gegenüber der aus dem Stand der Technik bekannten Substanz(C) zeigen bei diesem Test z.B. die Verbindungen gemäß folgender Herstellungsbeispiele: (I-1), (I-2), (I-3) und (I-5).

**Patentansprüche**

1. 1-Hydroxyethyl-azol-Derivate der allgemeinen Formel

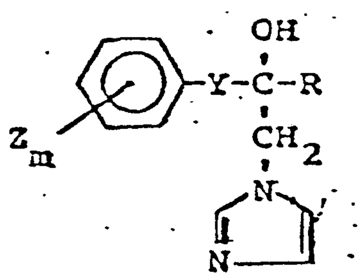

(I)

in welcher

R für tert.- Butyl, gegebenenfalls durch Methyl substituiertes Cyclopentyl oder für gegebenenfalls einfach oder zweifach, gleich oder verschieden durch Fluor, Chlor, Methyl oder Trifluormethyl substitiertes Phenyl steht,

Z für Fluor, Chlor, Brom, Methyl, tert.-Butyl, Cyclohexyl, Methoxy, Methylthio, Trifluormethyl, Trifluormethoxy, Trifluormethylthio oder für jeweils gegebenenfalls einfach oder zweifach, gleich oder verschieden durch Fluor, Chlor und/oder Methyl substituiertes Phenyl, Phenoxy, Benzyl oder Benzyloxy steht,

m für die Zahlen o, 1 oder 2 steht und

Y für die Gruppierungen $-CH_2-CH-_2$und $-O-CH-_2$steht, wobei im Falle der $-OCH_2$-Gruppe das Sauerstoffatom mit dem Phenylrest verbunden ist sowie deren Säureadditions-Salze und Metallsalz-Komplexe.

2. Verfahren zur Herstellung von 1-Hydroxyethyl-azol-. Derivaten der allgemeinen Formel

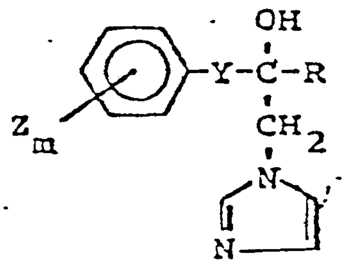

(I)

in welcher

R für tert.-Butyl, gegebenenfalls durch Methyl substituiertes Cyclopentyl oder für gegebenenfalls einfach oder zweifach, gleich oder verschieden durch Fluor, Chlor, Methyl oder Trifluormethyl substituiertes Phenyl steht,

Z für Fluor, Chlor, Brom, Methyl, tert.-Butyl, Cyclohexyl, Methoxy, Methylthio, Trifluormethyl, Trifluormethoxy, Trifluormethylthio oder für jeweils gegebenenfalls einfach oder zweifach, gleich oder verschieden durch Fluor, Chlor und/oder Methyl substituiertes Phenyl, Phenoxy, Benzyl oder Benzyloxy steht,

m für die Zahlen o, 1 oder 2 steht und

Y für die Gruppierungen $-CH_2-CH_2-$ und $-O-CH_2-$ steht, wobei im Falle der $-OCH_2$-Gruppe das Sauerstoffatom mit dem Phenylrest verbunden ist,

sowie deren Säureadditions-Salzen und Metallsalz-Komplexen,

dadurch gekennzeichnet, daß man Oxirane der Formel

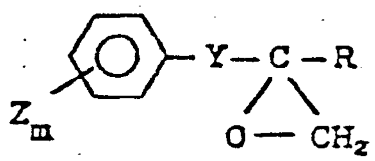

(II)

in welcher

R,Y, Z und m die oben angegebene Bedeutung haben, mit imidazol der Formel

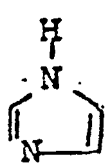

(III)

in Gegenwart eines Verdünnungsmittels und gegebenenfalls in Gegenwart einer Base unsetzt, und gegebenenfalls anschließend an die erhaltenen Verbindungen der Formel (I) eine Säure oder ein Metallsalz addiert.

3)Pflanzenwachstumsregulierende und fungizide Mittel gekennzeichnet durch einen Gehalt an mindestens einem 1-Hydroxyethyl-azol-Derivat der allgemeinen Formel (I) bzw. einem Säureadditions-Salz oder Metallsalz-Komplex eines 1-Hydroxyethyl-azol-Derivates der allgemeinen Formel (I) neben Streckmitteln und/oder oberflächenaktiven Stoffen.

4)Verwendung von 1-Hydroxyethyl-azol-Derivaten der allgemeinen Formel (I) bzw. Säureadditions-Salzen oder Metallsalz-Komplexen von 1-Hydroxyethyl-azol-Derivaten der allgemeinen Formel (I) zur Regulierung des Pflanzenwachstums bzw. zur Bekämpfung von Pilzen.

5)Verfahren zur Herstellung von pflanzenwachstumsregulierenden und fungiziden Mitteln, dadurch gekennzeichnet, daß man 1-Hydroxyethyl-azol-Derivate der allgemeinen Formel (I) bzw. Säureadditions-Salze oder Metallsalz-Komplexe von 1-Hydroxyethyl-azol-Derivaten der allgemeinen Formel (I) mit Streckmitteln und/oder oberflächenaktiven Stoffen vermischt.

**Claims**

1. 1-Hydroxyethyl-azole derivatives of the general formula

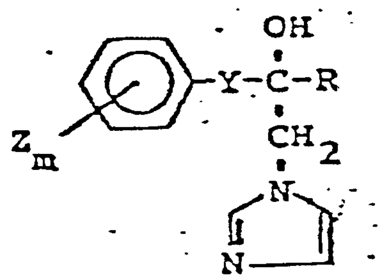

(I)

in which

R represents tert.-butyl, optionally methyl-substituted cyclopentyl or phenyl which is optionally mono- or disubstituted by identical or different substituents, these being fluorine, chlorine, methyl or trifluoromethyl,

Z represents fluorine, chlorine, bromine, methyl, tert.-butyl, cyclohexyl, methoxy, methylthio, trifluoromethyl, trifluoromethoxy, trifluoromethylthio, or phenyl, phenoxy, benzyl or benzyloxy, each of which is optionally mono- or disubstituted by identical or different substituents, these being fluorine, chlorine and/or methyl,

m represents the numbers 0, 1 or 2 and

Y represents the groupings $-CH_2-CH_2-$ and $-O-CH_2-$, the oxygen atom being linked with the phenyl radical in the case of the $-O-CH_2$ group,

and acid addition salts and metal salt complexes thereof.

2. Process for the preparation of 1-hydroxyethyl-azole derivatives of the general formula

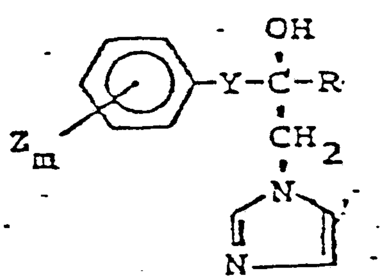

(I)

in which

R represents tert.-butyl, optionally methyl-substituted cyclopentyl or phenyl which is optionally mono- or disubstituted by identical or different substituents, these being fluorine, chlorine methyl or trifluoromethyl,

Z represents fluorine, chlorine, bromine, methyl,-tert.-butyl, cyclohexyl, methoxy, methylthio, trifluoromethyl, trifluoromethoxy, trifluoromethylthio or phenyl, phenoxy, benzyl or benzyloxy, each of which is optionally mono- or disubstituted by identical or different substituents, these being fluorine, chlorine and/or methyl,

m represents the numbers 0, 1 or 2 and

Y represents the groupings $-CH_2-CH_2-$ and $-O-CH_2-$, the oxygen atom being linked with the phenyl radical in the case of the $-OCH_2-$ group,

and acid addition salts and metal salt complexes thereof, characterised in that oxiranes of the formula

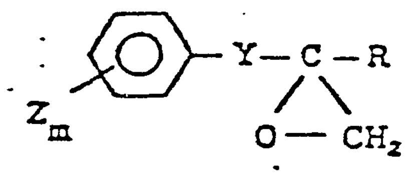

(II)

in which
R, Y, Z and m have the abovementioned meaning, are reacted with imidazole of the formula

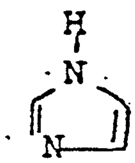

(III)

in the presence of a diluent and if appropriate in the presence of a base, and, if desired, an acid or a metal salt is then added on to the compounds of the formula (I) thus obtained.

3. Plant-growth-regulating and fungicidal agents, characterised in that they contain at least one 1-hydroxyethyl-azole derivative of the general formula (I) or at least one acid addition salt or metal salt complex of a 1-hydroxyethyl-azole derivative of the general formula (I) in addition to extenders and/or surface-active substances.

4. Use of 1-hydroxyethyl-azole derivatives of the general formula (I) or acid addition salts or metal salt complexes of 1-hydroxyethyl-azole derivatives of the general formula (I) for regulating plant growt or for combating fungi.

5. Process for the preparation of plant-growth-regulating and fungicidal agents, characterised in that 1-hydroxyethyl-azole derivatives of the general formula (I) or acid addition salts or metal salt complexes of 1-hydroxyethyl-azole derivatives of the general formula (I) are mixed with extenders and/or surface-active substances.

**Revendications**

1. Dérivés 1-hydroxyéthyl-azoliques de formule générale:

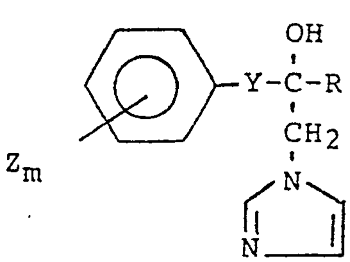

(I)

dans laquelle
R représente un t-butyle, un cyclopentyle éventuellement substitué par un méthyle ou un phényle éventuellement substitué une ou deux fois, de manière identique ou differente, par du fluor, chlore, méthyle ou trifluorométhyle,

Z du fluor, chlore, brome, méthyle, t-butyle, cyclohexyle, méthoxy, méthylthio, trifluorométhyle, trifluorométhoxy, trifluorométhylthio, ou bien un phényle, phénoxy, benzyle ou benzyloxy éventuellement substitués chacun une ou deux fois, de manière identique ou différente, par du fluor, chlore et/ou méthyle,

m représente les nombres 0, 1 ou 2 et

Y représente les groupements $-CH_2-CH_2-$ et -0-CH2-, dans le cas du groupe -OCH2- l'atome d'oxygène étant relié au radical phényle,

de même que leurs sels d'addition d'acides et complexes de sels métalliques.

2. Procédé de fabrication de dérivés 1-hydro-xyéthyl-azoliques de formule générale:

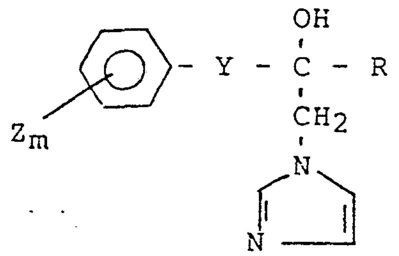

(I)

dans laquelle

R représente un t-butyle, un cyclopentyle éventuellement substitué par un méthyle, ou un phényle éventuellement substitué une ou deux fois, de manière identique ou differente, par du fluor, chlore, méthyle ou trifluorométhyle,

Z du fluor, chlore, brome, méthyle, t-butyle, cyclohexyle, méthoxy, méthylthio, trifluorométhyle, trifluorométhoxy, trifluorométhylthio ou bien un phényle, phénoxy, benzyle ou benzyloxy éventuellement substitués chacun une ou deux fois, de manière identique ou différente, par du fluor, chlore et/ou méthyle,

m représente les nombres 0, 1 ou 2 et

Y les groupements $-CH2-CH_2-$ et $-0-CH_2-$, dans le cas du groupe $-OCH_2-$ l'atome d'oxygène étant relié au radical phényle,

de même que de leurs sels d'addition d'acides et complexes de sels métalliques, caractérisé en ce qu'on fait réagir des oxiranes de formule:

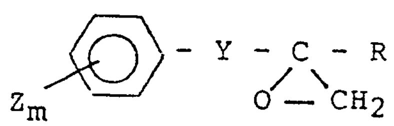

(II)

dans laquelle

R, Y, Z et m ont la signification indiquée plus haut, avec de l'imidazol de formule:

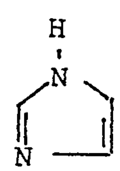

(III)

en présence d'un diluant et éventuellement en présence d'une base, et en ce que, le cas échéant, on fixe ensuite sur les composés obtenus de formule (I) un acide ou un sel métallique.

3. Agents régulateurs de croissance des plantes et fongicides, caractérisés par une teneur en au moins un dérivé 1-hydroxyéthyl-azolique de formule générale (I) ou en un sel d'addition d'acide ou complexe de sel métallique d'un dérivé 1-hydroxye-thyl-azolique de formule générale (I) à côté de diluants et/ou de substances tensioactives.

4. Utilisation de dérivés 1-hydroxyéthyl-azoliques de formule générale (I) ou de sels d'addition d'acides ou de

complexes de sels métalliques de dérivés 1-hydroxyéthyl-azoliques de formule générale (I) pour régler la croissance des plantes ou pour combattre les champignons.

5. Procédé de fabrication d'agents régulateurs de la croissance des plantes et fongicides, caractérisé en ce qu'on mélange des dérivés 1-hydroxyéthyl-azoli-ques de formule générale (I) ou des sels d'addition d'acides ou complexes de sels métalliques de dérivés 1-hydroxyéthyl-azoliques de formule générale (I) avec des diluants et/ou des substances tensioactives.